# EUROPEAN PATENT APPLICATION

(11) **EP 3 470 019 A1**
(43) Date of publication of application: **17.04.2019**
(21) Application number: 17810405.5
(22) Date of filing: 08.06.2017
(51) Int. Cl.: A61F 2/16

(54) **MONOFOCAL INTRAOCULAR LENS**

(30) Priority: 09.06.2016 JP 2016115696
(71) Applicant: Santen Pharmaceutical Co., Ltd., Higashiyodogawa-ku Osaka-shi Osaka 533-8651 (JP)
(72) Inventor: IKEDA, Yuji, Kyoto-shi Kyoto 606-0025 (JP); YAMADA, Yoshitaka, Osaka-shi Osaka 530-8552 (JP); NAKAJIMA, Mari, Osaka-shi Osaka 530-8552 (JP)
(74) Representative: Müller-Boré & Partner Patentanwälte PartG mbB
(86) International application number: PCT/JP2017/021359
(87) International publication number: WO 2017/213232

(57) **Abstract**

Provided is a monofocal intraocular lens that has larger depth of field than conventional monofocal intraocular lenses and that is capable of reducing the chances that patients who have received surgery will need to wear eyeglasses. A monofocal intraocular lens includes a lens part, the lens part having a base power and having the following MTF characteristics at 50 lp/mm with respect to a light beam passing through a region extending from a center of the lens part to a radius of 1.5 mm: a value of MTF is not less than 20% over a range of from 0 to +1.5 diopters from the base power; and a curve representing a diopter dependence of MTF, in which diopter is taken on a horizontal axis, has one local maximum value and does not have a local minimum value within the range of from 0 to +1.5 diopters from the base power where the value of MTF is not less than 20%.

## Description

### Technical Field

The present invention relates to a monofocal intraocular lens with a large depth of field.

### Background Art

Intraocular lenses for use after cataract surgery are known. An intraocular lens is inserted into an eyeball to substitute the function of the crystalline lens, which has been opacified by the progress of cataract, after the crystalline lens is removed by surgery. One of such intraocular lenses is a monofocal intraocular lens, which has only one focal point. The monofocal intraocular lens allows a user to see, with high resolution, an object positioned at a plane optically conjugate to the retina (for example, see Patent Literature 1).

### Citation List

### [Patent Literature]

[Patent Literature 1]
Japanese Patent Application Publication, Tokukai, No. 2011-41826

### Summary of Invention

### Technical Problem

However, a usual monofocal intraocular lens has only one pair of optically conjugate planes, and therefore, assuming that the image focus point resides on the retina, there is only one corresponding conjugate plane on the object side. Therefore, the depth of field, over which a high-resolution vision is available, is narrow, and thus a user is unable to obtain an area in which both near and far objects appear in acceptably sharp focus. The distance from the eye to an area that appears in acceptably sharp focus is referred to as the distance to field (or field distance). If the monofocal intraocular lens is designed such that distant objects are in focus, then near objects are out of focus, necessitating eyeglasses for near viewing. Similarly, if the monofocal intraocular lens is designed such that near objects are in focus, then distant objects are out of focus, necessitating eyeglasses for distance viewing. This should be inconvenient for patients with usual monofocal intraocular lenses.

The present application is to improve such a monofocal intraocular lens, and an object thereof is to provide a monofocal intraocular lens that has a larger depth of field than conventional monofocal intraocular lenses and that is capable of reducing the chances that patients who have received surgery will need to wear eyeglasses.

### Solution to Problem

In order to attain the above object, a monofocal intraocular lens in accordance with one aspect of the present invention includes a lens part, the lens part having a base power and having the following MTF characteristics at 50 lp/mm with respect to a light beam passing through a region extending from a center of the lens part to a radius of 1.5 mm: a value of modulation transfer function (MTF) is not less than 20% over a range of from 0 to +1.5 diopters from the base power; and a curve representing a diopter dependence of MTF, in which diopter is taken on a horizontal axis, has one local maximum value and does not have a local minimum value within the range of from 0 to +1.5 diopters from the base power where the value of MTF is not less than 20%.

### Advantageous Effects of Invention

The present invention brings about an effect of making it possible to provide a monofocal intraocular lens that is larger in clearly viewable range than conventional monofocal intraocular lenses and that is capable of reducing the chances that a user will need to wear eyeglasses.

### Brief Description of Drawings

Fig. 1 is a graph showing a relationship between diopter and MTF of a lens part (i.e., MTF characteristics) of a monofocal intraocular lens in accordance with the present embodiment, and also showing MTF characteristics of a conventional monofocal intraocular lens for comparison.
Fig. 2 shows perspective views each schematically illustrating the monofocal intraocular lens in accordance with the present embodiment. (a) of Fig. 2 is a perspective view of the monofocal intraocular lens as viewed obliquely from above, and (b) of Fig. 2 is a perspective view of the monofocal intraocular lens as viewed obliquely from below.
Fig. 3 is a graph showing a relationship between the distance from the center of a lens part and diopter of the lens part of the monofocal intraocular lens in accordance with the present embodiment.

### Description of Embodiments

A monofocal intraocular lens in accordance with the present invention includes a lens part, the lens part having a base power and having the following MTF characteristics at 50 lp/mm in a region extending from the center of the lens part to a radius of 1.5 mm: a value of MTF is not less than 20% over a range of from 0 to +1.5 diopters from the base power; and a curve representing a diopter dependence of MTF, in which diopter is taken on a horizontal axis, has one local maximum value and does not have a local minimum value within the range of from 0 to +1.5 diopters from the base power where the value of MTF is not less than 20%.

The above configuration makes it possible to improve a user's vision to the extent that the user does not need to wear eyeglasses to identify the faces and facial expressions of people in the distance range of from infinity to about 70 cm in front of the eye, despite being a monofocal intraocular lens. This makes it possible to provide a monofocal intraocular lens that is larger in depth of field, over which clear vision is obtained, than conventional monofocal intraocular lenses and that is capable of reducing the chances that the user will need to wear eyeglasses.

In the above case, the local maximum value of the curve representing the diopter dependence of MTF corresponds to the visual acuity at the field distance at which vision is clearest in the range of from infinity to about 70 cm in front of the eye. By ensuring that the value of MTF at the position at which the highest resolution is achieved is not less than 45%, it is possible to achieve improved ease of viewing. By ensuring that the value of MTF at the position at which the highest resolution is achieved is not less than 50%, not less than 55%, it is possible to achieve further improved ease of viewing.

The following description will discuss an embodiment of the present invention in detail. In the following description, a best mode is discussed, in which the local maximum value (MTF at base power) of a curve representing the diopter dependence of MTF reaches about 60%.

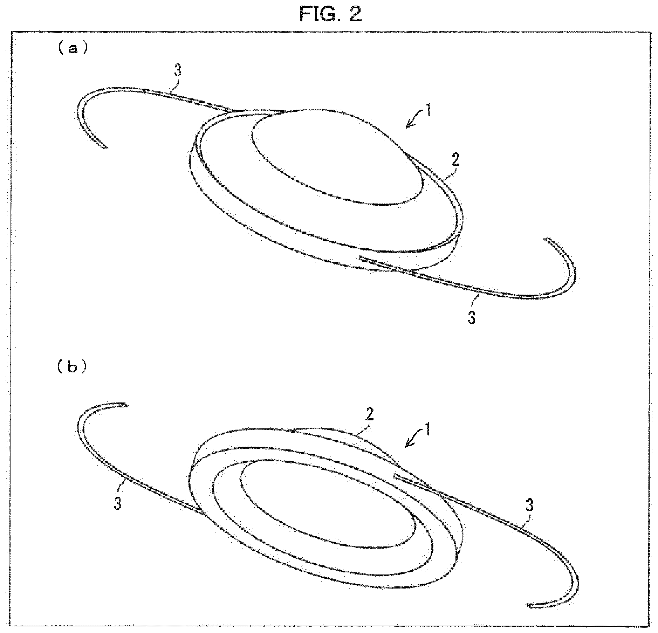

Fig. 2 shows perspective views each schematically illustrating a monofocal intraocular lens 1 in accordance with one embodiment of the present invention. (a) of Fig. 2 is a perspective view of the monofocal intraocular lens 1 as viewed obliquely from above, and (b) of Fig. 2 is a perspective view of the monofocal intraocular lens 1 as viewed obliquely from below.

The monofocal intraocular lens 1 includes: a lens part 2, which is a lens body; and two securing members 3 configured to secure the monofocal intraocular lens 1 within an eyeball. A material for the lens part 2 can be, for example, a crosslinked acrylate copolymer that is in major use today and that is capable of being folded when the intraocular lens is implanted, or the like. The material for the lens part 2 is not limited to a particular kind, and may alternatively be, for example, polymethyl methacrylate, a silicone elastomer composed mainly of polydimethylsiloxane, or the like. The material for the lens part 2 is more preferably a flexible (deformable) material such that the lens part can be rolled or folded to be passed through a small cut in the eyeball, in terms of achieving less invasive surgery.

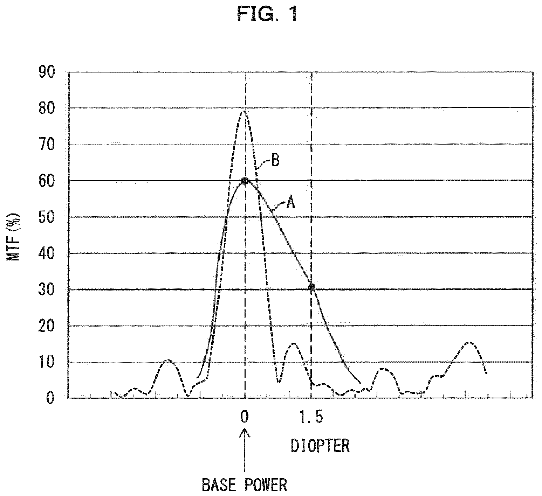

Fig. 1 is a graph showing a relationship between diopter (corresponding to field distance) and MTF of the lens part 2 (i.e., MTF characteristics) of the monofocal intraocular lens 1 in accordance with the present embodiment, and also showing MTF characteristics of a conventional monofocal intraocular lens for comparison. In Fig. 1, the horizontal axis shows diopter, whereas the vertical axis shows MTF determined at 50 lp/mm using a light beam passing through a region extending from the center of the lens part 2 to a radius of 1.5 mm. Solid line A represents the MTF characteristics of the monofocal intraocular lens 1 in accordance with the present embodiment, whereas dotted line B represents the MTF characteristics of the conventional monofocal intraocular lens. Both the monofocal intraocular lens 1 in accordance with the present embodiment and the conventional monofocal intraocular lens have a base power of zero diopters.

As is clear from line B, the MTF of the conventional monofocal intraocular lens at the base power reaches about 80%. The value of the MTF sharply decreases with deviation from the base power. With the conventional monofocal intraocular lens having such MTF characteristics, the clearly viewable range, over which the MTF is not less than 20% and in which a user is able to identify the faces and facial expressions of people, is a narrow range of from infinity to about 2 m in front of the eye. In in a region nearer than 2 m, the user is unable to identify the faces and facial expressions of people without eyeglasses.

In contrast, as is clear from line A, the monofocal intraocular lens 1 in accordance with the present embodiment has, at the base power, one local maximum value of MTF that reaches about 60%. The value of the MTF sharply decreases with deviation from the base power in the negative direction (to the left along the horizontal axis), but the value of the MTF moderately decreases with deviation from the base power in the positive direction (to the right along the horizontal axis) as diopter increases. Thus, the monofocal intraocular lens 1 in accordance with the present embodiment maintains a value of MTF of 30% even at a position deviated from the base power by about +1.5 diopters.

With the monofocal intraocular lens 1 in accordance with the present embodiment having such MTF characteristics, the clearly viewable range, over which the MTF is not less than 20%, is a wide range of from infinity to about 70 cm in front of the eye. Although the ease of viewing gradually decreases from distance to near, the user is able to identify the faces and facial expressions of people without eyeglasses in the range of up to about 70 cm in front of the eye. Note that, in order to obtain clear vision of objects nearer than 70 cm, it is only necessary to wear eyeglasses for near viewing.

As such, the monofocal intraocular lens 1 in accordance with the present embodiment is wider in clearly viewable range than the conventional monofocal lenses, and thus is capable of reducing the chances that the user will need to wear eyeglasses.

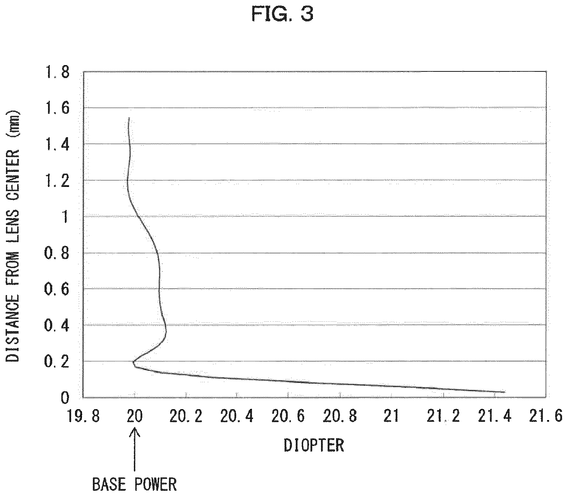

Fig. 3 is a graph showing, for a group of circular marginal rays passing through the lens part 2, a relationship between the distance from the center of the lens part 2 and diopter (field distance) of the lens part 2 of the monofocal intraocular lens 1 in accordance with the present embodiment. The horizontal axis in the graph shows diopter, whereas the vertical axis in the graph shows the distance from the center of the group of marginal rays passing through the lens part 2. The base power is 20 diopters.

As shown in Fig. 3, within the region extending from the center of the lens part 2 to a radius of 0.2 mm, the field distance continuously changes from [+1.5 diopters from the base power] (i.e., 21.5 diopters) to the base power (i.e., 20 diopters) as the distance between the group of rays and the center of the lens part 2 increases. In a case of a group of rays passing through an outer region outside the region extending from the center of the lens part 2 to a radius of 0.2 mm, the field distance fluctuates within the range of not greater than [+0.2 diopters from the base power] (i.e., 20.2 diopters). Note that Fig. 3 only shows data for the area extending from the center to a radius of 1.5 mm (corresponding to the radius of pupil size under the condition of illumination intensity of daylight); however, also in the outer area outside the area extending from the center to a radius of 1.5 mm, the field distance fluctuates within the range of not greater than [+0.2 diopters from the base power] (i.e., 20.2 diopters).

According to the above arrangement, the outer area of the lens part outside the inner area extending from the center of the lens part to a radius of 1.5 mm may be arranged such that the angle of refraction of the outer area is smaller than the angle of refraction of a lens shape determined by extrapolation of the shape of the inner area of the lens part so that the outer area is suitable for distance viewing, and thereby a larger depth of field can be obtained. In the opposite case where the outer area of the lens part is arranged such that the angle of refraction of the outer area is greater than the angle of refraction of the lens shape determined by extrapolation of the shape of the inner area of the lens part, it is possible to allow a group of rays, which is emitted from infinity or a sufficiently distant object and would otherwise be focused behind the retina, to converge on a point at which an object located within the distance range of from infinity to about 70 cm in front of the eye (this range corresponds to the range of from +1.5 to 0 diopters from the base power) is supposed to be focused. This makes it possible to improve the overall MTF values even in darkness where the pupil dilates.

Furthermore, since the MTF dependence curve is a curve with no discontinuous points or lines when the diopter is taken on the horizontal axis, the surface profile of the corresponding lens necessarily forms a continuous curve. Such an intraocular lens with no discontinuous points or lines also has the effect of making it possible to reduce the following phenomena: glare phenomenon, in which highlights look glaring; halo phenomenon, in which a ring appears around light; and the like, each of which is one of visual disturbances that would occur after the lens is implanted.

Note that Fig. 3 serves merely as an example. Assuming that the distance from the center of the lens part 2 to the extremity of a region, over which the field distance continuously changes from +1.5 to 0 diopters from the base power, of the lens part 2 is referred to as a first radius, the first radius may be any radius falling within the range of from 0.1 to 0.3 mm. If the first radius is less than 0.1 mm, it is not possible to ensure a necessary level of MTF in an intermediate field distance range of up to about 70 cm. On the other hand, if the first radius is greater than 0.3 mm, it is not possible to ensure a necessary level of MTF in a distant range. When the first radius is 0.2 mm, it is possible to effectively ensure a necessary level of MTF both in the intermediate and distant ranges.

Similarly, assume that the distance from the center of the lens part 2 to the periphery of a region, which extends outward from the first radius and in which the field distance fluctuates within the range of not greater than +0.2 diopters from the base power, is referred to as a second radius. In this case, the second radius is preferably equivalent to the radius of pupil size under the condition of illumination intensity of daylight. Although the second radius in Fig. 3 is 1.5 mm, this does not imply any limitation. Since the normal pupil size under the condition of illumination intensity of daylight is 2.5 to 4.0 mm, the second radius may be any radius falling within the range of from 1.25 to 2.0 mm.

If the second radius is less than 1.25 mm, the MTF dependence curve, when diopter is taken on the horizontal axis, may have some discontinuous point or line within a range corresponding to the pupil size under the condition of illumination intensity of daylight, and it is not possible to obtain the effect of reducing the foregoing glare phenomenon, halo phenomenon, and the like. When the second radius is 1.5 mm, in a case of a pupil size of 3.0 mm under the condition of illumination intensity of daylight, it is possible to effectively reduce the foregoing glare phenomenon, halo phenomenon, and the like.

### [Recap]

In order to attain the foregoing object, a monofocal intraocular lens in accordance with one aspect of the present invention includes a lens part, the lens part having a base power and having the following modulation transfer function (MTF) characteristics at 50 lp/mm with respect to a light beam passing through a region extending from a center of the lens part to a radius of 1.5 mm: a value of MTF is not less than 20% over a range of from 0 to +1.5 diopters from the base power; and a curve representing a diopter dependence of MTF, in which diopter is taken on a horizontal axis, has one local maximum value and does not have a local minimum value within the range of from 0 to +1.5 diopters from the base power where the value of MTF is not less than 20%.

According to the above configuration, the MTF characteristics at 50 lp/mm in the region extending from the center of the lens part to a radius of 1.5 mm, that is, in the region equivalent to pupil size under the condition of illumination intensity of daylight, are such that the curve representing the diopter dependence of MTF, in which diopter is taken on the horizontal axis, has one local maximum value and does not have a local minimum value within the range of from 0 to +1.5 diopters from the base power where the value of MTF is not less than 20%. Specifically, the number of conjugate planes on the object side of this lens, assuming that the image focus point resides on the retina, is one, which means that this lens is undoubtedly a monofocal intraocular lens. Furthermore, according to the above configuration, in the region extending from the center of the lens part to a radius of 1.5 mm, the value of MTF at 50 lp/mm is not less than 20% over the range of from 0 to +1.5 diopters from the base power. That is, the monofocal intraocular lens has a value of MTF of not less than 20% within the distance range of from infinity to about 70 cm in front of the eye, which corresponds to the above range of from 0 to +1.5 diopters from the base power.

MTF is an indicator concerning the resolution of the eyes, and can be expressed as so-called visual acuity. Generally, provided that the value of MTF is not less than 20%, the visual acuity is good enough to allow faces and facial expressions of people to be identified. That is, according to the above configuration, in the distance range of from infinity to about 70 cm in front of the eye, a user is able to identify the faces and facial expressions of people without wearing eyeglasses.

As such, the monofocal intraocular lens in accordance with one aspect of the present invention is larger in clearly viewable range than conventional monofocal intraocular lenses, and achieves a clear vision without eyeglasses in the distance range of from infinity to about 70 cm in front of the eye.

The monofocal intraocular lens in accordance with one aspect of the present invention is preferably arranged such that the local maximum value of MTF is not less than 45%, more preferably not less than 50%, even more preferably not less than 55%. The local maximum value is equivalent to the visual acuity at the field distance at which the resolution is highest in the field distance range of from infinity to about 70 cm in front of the eye. By ensuring that the value of MTF is not less than 45% at the field distance at which the resolution is highest, it is possible to improve ease of viewing. By ensuring that the value of MTF is not less than 50%, not less than 55%, at the field distance at which the resolution is highest, it is possible to further improve ease of viewing.

The monofocal intraocular lens in accordance with one aspect of the present invention is preferably arranged such that the lens part is such that: a field distance for a first group of circular marginal rays, of rays passing through a region extending from the center of the lens part to a first radius, changes continuously from +1.5 to 0 diopters from the base power with increasing distance from the center of the lens part; a field distance for a second group of circular marginal rays passing through a region extending outward from the first radius to at least a second radius is not greater than +0.2 diopters from the base power; and a curve representing field distance (diopter)'s dependence on a height of marginal rays from the center of the lens part has no discontinuities over a region extending from the center of the lens part to at least the second radius.

In other words, it is preferable that: a group of so-called paraxial rays, which come from a field distance corresponding to +1.5 diopters from the base power and pass through the center of the lens part or positions very close to the center of the lens part, converge on the retina; a field distance for rays passing through a region having the first radius continuously changes from +1.5 to 0 diopters from the base power with increasing distance from the center; and a curve for circular marginal rays passing through the lens, in which field distance is taken on the horizontal axis and the distance from the center of the lens part is taken on the vertical axis, is a curve with no discontinuities.

According to the above arrangement, the field distance for rays within the first radius from the center of the lens part continuously changes from +1.5 to 0 diopters from the base power with increasing distance from the center. That is, the rays passing through a region extending from the center of the lens part to the first radius corresponds to rays from what is called an intermediate distance range, which is a range of field distances of about 70 cm, being focused on the retina. Furthermore, rays passing through a region extending outward from the first radius to at least the second radius from the center of the lens part corresponds to rays from a far distance range, that is, from not greater than +0.2 diopters from the base power, being focused on the retina.

In addition to the above arrangement, an outer area of the lens part outside a region extending from the center of the lens part to the second radius may be arranged such that the angle of refraction of the outer area is smaller than the angle of refraction of a lens shape determined by extrapolation of the shape of the inner area of the lens part so that the outer area is suitable for distance viewing, and thereby a larger depth of field can be obtained. In the opposite case where the outer area of the lens part is arranged such that the angle of refraction of the outer area is greater than the angle of refraction of the lens shape determined by extrapolation of the shape of the inner area of the lens part, it is possible to allow a group of rays, which is emitted from infinity or a sufficiently distant object and would otherwise be focused behind the retina, to converge on a point at which an object located within the distance range of from infinity to about 70 cm in front of the eye (this range corresponds to the range of from +1.5 to 0 diopters from the base power) is supposed to be focused. This makes it possible to improve the overall MTF values even in darkness where the pupil dilates to a greater extent.

Furthermore, according to the above arrangement, since the MTF dependence curve is a curve with no discontinuous points or lines when the diopter is taken on the horizontal axis, the surface profile of the corresponding lens necessarily forms a continuous curve. Such an intraocular lens with no discontinuous points or lines also has the effects of making it possible to reduce the following phenomena: glare phenomenon, in which highlights look glaring; halo phenomenon, in which a ring appears around light; and the like, each of which is one of visual disturbances that would occur after the lens is implanted.

The monofocal intraocular lens in accordance with one aspect of the present invention can be arranged such that the first radius is 0.1 to 0.3 mm or is 0.2 mm. The monofocal intraocular lens in accordance with one aspect of the present invention can be arranged such that the second radius is a distance corresponding to a radius of pupil size under a condition of illumination intensity of daylight, which can be 1.25 to 2.0 mm, or can be 1.5 mm.

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

- 1: Monofocal intraocular lens
- 2: Lens part
- 3: Securing member

## Claims

1. A monofocal intraocular lens comprising a lens part, the lens part having a base power and having the following MTF characteristics at 50 lp/mm with respect to a light beam passing through a region extending from a center of the lens part to a radius of 1.5 mm:
a value of MTF is not less than 20% over a range of from 0 to +1.5 diopters from the base power; and
a curve representing a diopter dependence of MTF, in which diopter is taken on a horizontal axis, has one local maximum value and does not have a local minimum value within the range of from 0 to +1.5 diopters from the base power where the value of MTF is not less than 20%.

2. The monofocal intraocular lens according to claim 1, wherein the local maximum value of MTF is not less than 45%.

3. The monofocal intraocular lens according to claim 1, wherein the local maximum value of MTF is not less than 50%.

4. The monofocal intraocular lens according to claim 1, wherein the local maximum value of MTF is not less than 55%.

5. The monofocal intraocular lens according to any one of claims 1 to 4, wherein the lens part is such that:
a field distance for a first group of circular marginal rays, of rays passing through a region extending from the center of the lens part to a first radius, changes continuously from +1.5 to 0 diopters from the base power with increasing distance from the center of the lens part;
a field distance for a second group of circular marginal rays passing through a region extending outward from the first radius to at least a second radius is not greater than +0.2 diopters from the base power; and
a curve representing field distance's dependence on a height of marginal rays from the center of the lens part has no discontinuities over a region extending from the center of the lens part to at least the second radius.

6. The monofocal intraocular lens according to claim 5, wherein the first radius is 0.1 to 0.3 mm.

7. The monofocal intraocular lens according to claim 5 or 6, wherein the first radius is 0.2 mm.

8. The monofocal intraocular lens according to any one of claims 5 to 7, wherein the second radius is a distance corresponding to a radius of pupil size under a condition of illumination intensity of daylight.

9. The monofocal intraocular lens according to any one of claims 5 to 8, wherein the second radius is 1.25 to 2.0 mm.

10. The monofocal intraocular lens according to any one of claims 5 to 9, wherein the second radius is 1.5 mm.
